# EUROPÄISCHE PATENTSCHRIFT

(11) **EP 1 562 093 B1**
(45) Veröffentlichungstag und Bekanntmachung des Hinweises auf die Patenterteilung: **29.04.2015**
(21) Anmeldenummer: 05001528.8
(22) Anmeldetag: 26.01.2005
(51) Int. Cl.: G05D 7/06, A61M 15/00

(54) **Dosiervorrichtung**
Dosing device
Dispositif de dosage

(30) Priorität: 05.02.2004 DE 102004006453
(43) Veröffentlichungstag der Anmeldung: 10.08.2005
(73) Patentinhaber: Aptar Radolfzell GmbH, 78315 Radolfzell (DE)
(72) Erfinder: Körner, Joachim, 88690 Uhldingen (DE); Helmlinger, Michael, 78315 Radolfzell (DE); Schürle, Holger, 78315 Radolfzell (DE)
(74) Vertreter: Patentanwälte Ruff, Wilhelm, Beier, Dauster & Partner

(56) Entgegenhaltungen:
- WO-A-86/05993
- WO-A-92/11050
- WO-A-02/070047
- US-A1- 2003 168 057

## Beschreibung

Die Erfindung betrifft eine Dosiervorrichtung nach dem Oberbegriff von Anspruch 1.

Dosiervorrichtungen sind in vielfältigen Ausführungsformen aus dem Stand der Technik bekannt. Sie werden zum Dosieren und Austragen von flüssigen oder pulverförmigen Medien im Bereich der Kosmetik und der Pharmazie eingesetzt. Dazu weisen bekannte Dosiervorrichtungen einen Mediumspeicher auf, in dem das auszutragende Medium bis zu einem Austragvorgang gespeichert ist. Weiterhin ist wenigstens ein Mediumkanal vorgesehen, der eine Förderung des Mediums vom Mediumspeicher hin zu einer Austragöffnung erlaubt. Das auszutragende Medium wird durch eine Druckbeaufschlagung durch die Austragöffnung gepresst und in eine Umgebung der Dosiervorrichtung abgegeben.

Zur Erfassung und Steuerung der Flüssigkeitsmenge, die ausgetragen wird, schlagen die Dokumente WO 02/070047 A1 und US 2003/0168057 A1 vor, durch Sensoren den Flüssigkeitsstrom vor dessen Austrag zur überwachen.

Die der Erfindung zugrunde liegende Aufgabe besteht darin, eine Dosiervorrichtung der eingangs genannten Art zu schaffen, die einen präziseren Austragvorgang ermöglicht.

Diese Aufgabe wird durch eine Dosiervorrichtung nach Anspruch 1 gelöst.

Eine Regeleinheit ermöglicht eine Regelung einer Stelleinrichtung durch Vergleich und Beeinflussung von Istwerten eines Mediumstroms anhand von Sollwerten für den Mediumstrom, die in einem Speicher abgelegt sind. Dabei werden die Istwerte des Mediumstroms gemäß den vorgegebenen Sollwerten beeinflusst. Dazu wird während des Austragvorganges dauerhaft oder zumindest zeitweilig ein Vergleich zwischen Soll- und Istwerten vorgenommen und ein aus dem Vergleich resultierendes Steuersignal an die Stelleinrichtung abgegeben. Das Steuersignal führt in der Stelleinrichtung zu einer Verringerung oder Vergrößerung des Mediumstroms. Die Stelleinrichtung kann dabei insbesondere als hydraulisches oder pneumatisches Steuerventil und/oder als Druckerzeuger oder Druckminderer ausgelegt sein.

Als Messeinrichtung kommen insbesondere induktive, kapazitive, optische oder mechanische Volumenstrommesser in Frage. Weiterhin kann zum Zweck der Volumenstrommessung auch eine Differenzdruckmesseinrichtung mit Messblende oder ein insbesondere optoelektrisch abgetasteter Schwimmkörper vorgesehen werden. Als Speicher für die Sollwerte kann insbesondere ein elektrischer, elektronischer oder mechanischer Speicher vorgesehen werden. Als Vergleicher kommen ebenfalls elektrische, elektronische oder mechanische Vergleichseinrichtungen in Frage. Durch die Regeleinheit kann insbesondere eine voreingestellte Mediummenge ausgetragen werden, wobei die Voreinstellung starr oder durch einen Benutzer verstellbar vorgesehen sein kann.

Die Anbringung einer Messeinrichtung stromabwärts nach der Austragöffnung ermöglicht eine Ermittlung der tatsächlich durch die Austragöffnung in die Umgebung abgegebenen Mediummenge.

In Ausgestaltung der Erfindung ist an dem Mediumkanal ein Dosierraum zur zumindest zeitweisen Aufnahme einer Flüssigkeitsmenge vorgesehen, dem die wenigstens eine Austragöffnung zugeordnet ist. Dabei ist der Dosierraum mit einer Vibrationseinheit versehen, die wenigstens mit einer Begrenzungsfläche des Dosierraums in Wirkverbindung steht, um diese für einen Austragvorgang in Schwingungen zu versetzen. Eine mit der Vibrationseinheit verbundene Abgabefunktionseinheit ist zur Aktivierung der Vibrationseinheit während einer Abgabezeitdauer vorgesehen. Ein Dosierraum wird durch mehrere Begrenzungsflächen definiert, wobei die Vibrationseinheit an zumindest einer Begrenzungsfläche vorgesehen ist. Die Vibrationseinheit, die insbesondere als Piezoschwinger ausgeführt ist, kann durch Beaufschlagung mit einer elektrischen Spannung die Begrenzungsfläche in Schwingungen versetzen. Für einen Austragvorgang wird Medium insbesondere über eine Fördereinrichtung wie eine manuell oder elektromechanisch betätigte Pumpeinrichtung, durch Öffnen eines Ventils an einem druckbeaufschlagten Mediumspeicher oder bei geeigneter Gestaltung des Mediumkanals durch Kapillarkräfte aus einem drucklosen Mediumspeicher in den Dosierraum eingebracht. Im Dosierraum herrscht Atmosphärendruck. Eine solche Ausgestaltung eignet sich insbesondere für den Einsatz bei einer Mikrodosiervorrichtung für die hochgenaue Dosierung kleinster Flüssigkeitsmengen. Ein Einsatz ist insbesondere im pharmazeutischen Bereich vorgesehen.

Dem Dosierraum ist an einer Begrenzungsfläche eine Austragöffnung zugeordnet, die das Medium nur dann passieren kann, wenn ein insbesondere durch eine Geometrie der Austragöffnung, eine Oberflächenbeschaffenheit der Begrenzungsfläche sowie eine Oberflächenspannung bzw. eine Viskosität des Mediums bestimmter Strömungswiderstand überwunden werden kann. Um auf das Medium im Dosierraum einen entsprechenden Druck aufzubringen, der zur Überwindung des Strömungswiderstandes führt, ist die Vibrationseinheit vorgesehen, die das im Dosierraum befindliche Medium mit Druckwellen beaufschlagt, so dass zumindest zeitweilig ein Mediumaustrag aus der Austragöffnung erfolgt. Für eine Aktivierung der Vibrationseinheit ist eine Abgabefunktionseinheit vorgesehen, die ein insbesondere zeitabhängiges elektrisches Signal an die Vibrationseinheit abgibt.

In weiterer Ausgestaltung der Erfindung ist die Abgabefunktionseinheit mit der Regeleinheit zur kontrollierten Mediumabgabe gekoppelt. Durch eine Kopplung der Abgabefunktionseinheit mit der Regeleinheit lässt sich eine Synchronisation zwischen dem Ist- und dem Sollwert des Mediumstroms und der Aktivierung der Vibrationseinheit herstellen. Damit kann erreicht werden, dass die Abgabefunktionseinheit nur so lange entsprechende Ansteuersignale an die Vibrationseinheit sendet, solange dies für die Erreichung des Sollwertes des Mediumstroms notwendig ist bzw. solange der Ist-Wert des Mediumstroms dem Soll-Wert entspricht.

In weiterer Ausgestaltung der Erfindung ist die Messeinrichtung in Fließrichtung des Mediums stromabwärts nach der Austragöffnung an dem Dosierraum angeordnet. Das Medium wird bedingt durch Druckkräfte, die von Fördereinrichtungen und insbesondere von der am Dosierraum vorgesehenen Vibrationseinheit aufgebracht werden, mit einem Druck beaufschlagt und kann entgegen dem Strömungswiderstand der Austragöffnung in die Umgebung der Dosiervorrichtung abgegeben werden. Dabei nimmt das Medium eine eindeutig definierte Fließrichtung ein, die vom Mediumspeicher über die Fördereinrichtung, den Dosierraum und die Austragöffnung in die Umgebung weist.

In weiterer Ausgestaltung der Erfindung weist die Messeinrichtung eine optische und/oder kapazitive und/oder induktive Sensoranordnung auf. Für eine optische Sensoranordnung, die in Form einer Lichtschranke oder eines Reflektionsmessers ausgeführt werden kann, wird von einer Lichtquelle ein Lichtsignal, insbesondere nahezu parallel zur einer Erstreckungsebene der mit der Austragsöffnung versehenen Begrenzungsfläche, ausgesandt. Anschließend kann mittels eines lichtempfindlichen Sensors entweder eine Analyse des transmittierten oder des reflektierten Lichtstrahls vorgenommen werden, wodurch sich ein Meßsignal ermitteln läßt, das Auskunft über eine Anzahl und/oder eine Dichte von Flüssigkeitsteilchen in einer definierten Meßstrecke gibt. Alternativ oder ergänzend ist auch eine Gestaltung der Meßeinrichtung als Ladungsmesser denkbar. Dabei werden ausgetragene Flüssigkeitsteilchen durch eine elektrisch vorgespannte Begrenzungsfläche mit einer Ladung versehen. Insbesondere kann jedem Flüssigkeitsteilchen genau eine Ladungseinheit zugeordnet werden oder eine größenporportionale Ladung mitgegeben werden. Durch die Aufstellung einer Ladungsbilanz kann dann eine Anzahl abgeströmter Ladungen ermittelt werden und somit auf die Anzahl der ausgetragenen Flüssigkeitsteilchen rückgeschlossen werden.

In weiterer Ausgestaltung der Erfindung ist der Mediumspeicher druckbeaufschlagt. Eine Druckbeaufschlagung des Mediumspeichers kann insbesondere durch ein im Mediumspeicher vorgesehenes Treibmittel in gasförmiger oder flüssiger Form und/oder durch eine mechanische Vorspannung einer deformierbaren oder beweglichen Wandung des Mediumspeichers verwirklicht werden. Die Verwendung von Treibmitteln, insbesondere von Treibgasen wie Kohlendioxid erlaubt eine besonders einfache mechanische Gestaltung des Mediumspeichers, da keine beweglichen Teile zur Druckbeaufschlagung des Mediums notwendig sind. Eine mechanische Druckbeaufschlagung hingegen erlaubt auch bei unterschiedlichsten Umweltgegebenheiten wie Temperaturschwankungen einen zuverlässigen Austrag des im Mediumspeicher vorgesehenen Mediums. Ein druckbeaufschlagter Mediumspeicher hat weiterhin den Vorteil, dass bei Öffnen eines Dosierventils ein im wesentlichen kontinuierlicher Mediumstrom aus dem Mediumspeicher gewährleistet werden kann.

In einer Weiterbildung der Erfindung ist vorgesehen, dass in dem Mediumkanal und/oder dem Mediumspeicher ein Gasabscheider vorgesehen ist. Ein Gasabscheider kann insbesondere als semipermeable Membran ausgeführt werden, die nur eindeutig definierte Bestandteile des Mediums in eine Umgebung austreten lässt, während andere Bestandteile des Mediums zurückgehalten werden. Die Anwendung eines derartigen Gasabscheiders ist insbesondere dann vorzusehen, wenn eine besonders präzise Dosierung angestrebt ist, bei der ein gasfreier Austrag des Mediums vorgenommen werden soll. Andere im Mediumspeicher befindliche Anteile, die eine Volumenstrommessung oder Volumenmessung behindern würden, können nicht durch die semipermeable Membran hindurchtreten und werden somit nicht ausgetragen. Bei derartigen unerwünschten Anteilen kann es sich insbesondere um im Medium gelöste Gasteilchen handeln, die zu Fehlmessungen der Messeinrichtungen und/oder zu einer Bildung von Gasblasen im Dosierraum und dadurch insbesondere zu einer Verringerung einer Austragseffizienz und/oder einer Austragsqualität führen können.

In weiterer Ausgestaltung der Erfindung ist an dem Mediumspeicher und/oder dem Gasabscheider ein Gasauslaß vorgesehen. Ein Gasauslaß kann insbesondere als hydrophober Membranfilter ausgeführt werden und dient zur Abscheidung gasförmiger Bestandteile aus dem flüssigen Medium. Der Gasauslaß kann dabei permanent oder in Abhängigkeit von Steuerbefehlen nur zeitweilig für das Austreten von Gas aus dem Medium freigeschaltet werden.

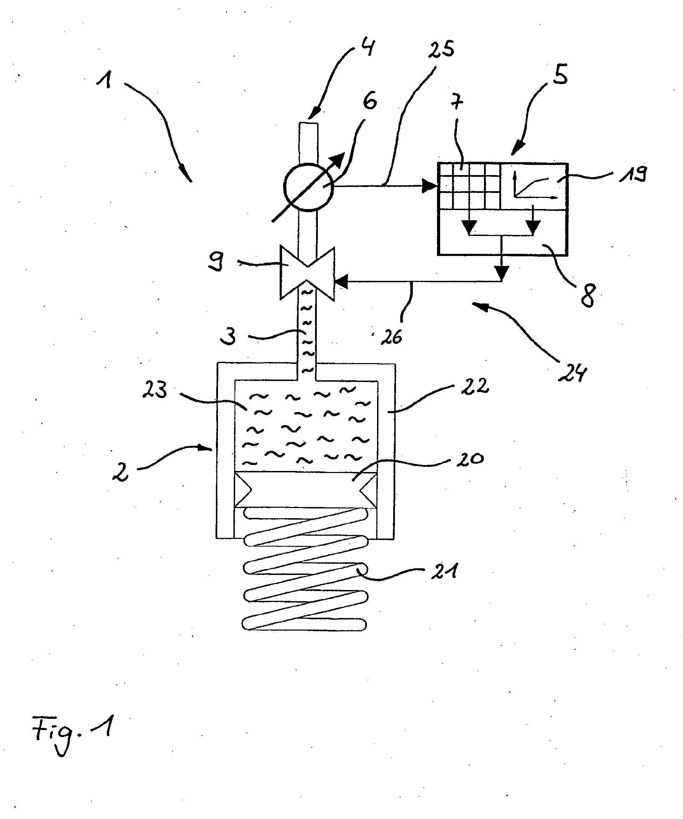

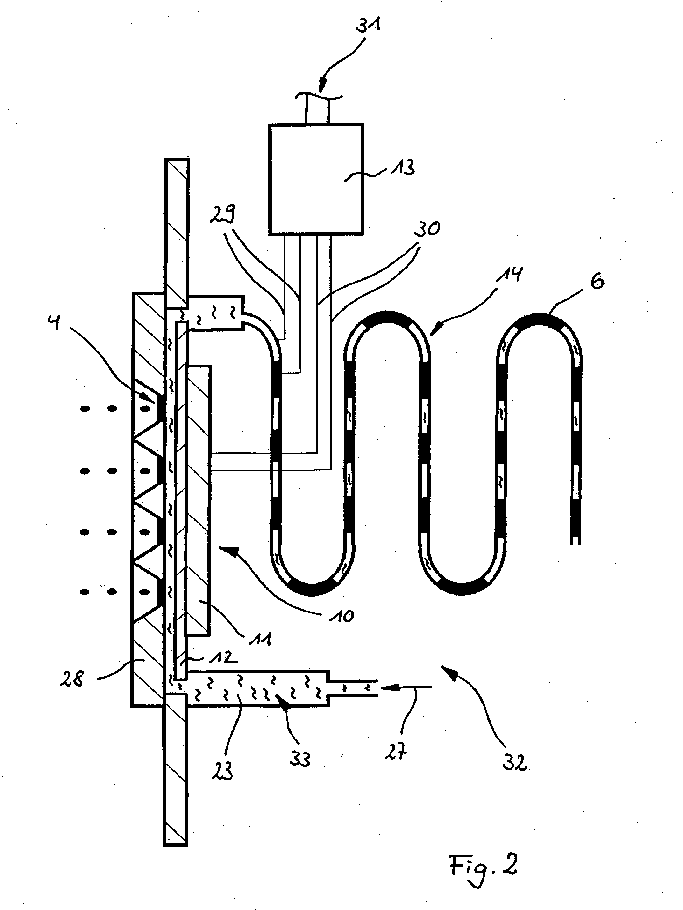

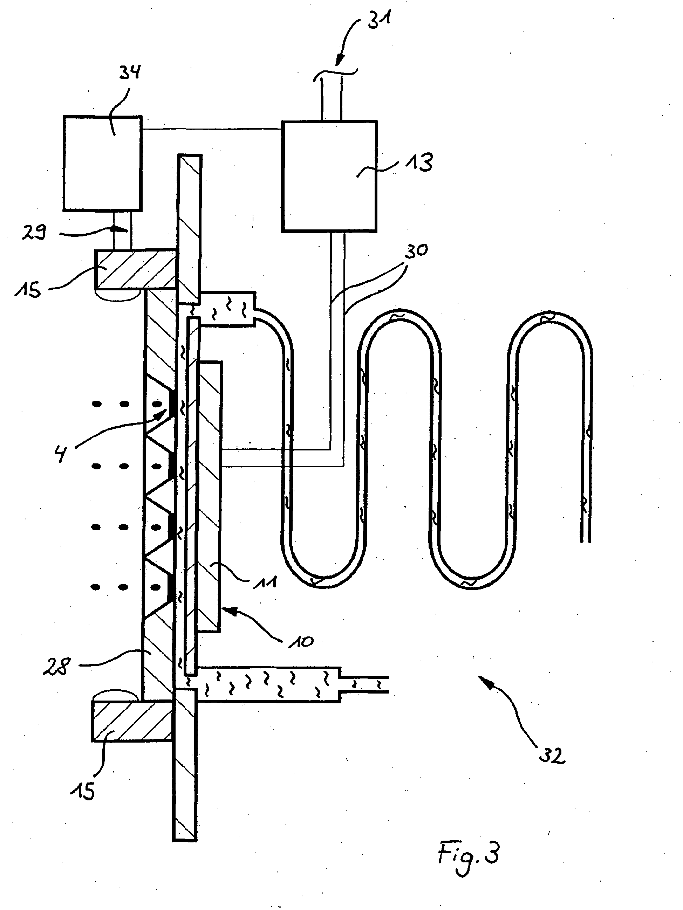

Weitere Vorteile und Merkmale der Erfindung ergeben sich aus den Ansprüchen sowie aus der nachfolgenden Beschreibung eines bevorzugten Ausführungsbeispiels der Erfindung, das anhand der Figur 3 dargestellt ist. Die anderen Figuren 1 und 2 stellen nicht von der Erfindung umfasste Ausgestaltungen dar, die nur zum besseren Verständnis der Ausführungsform der Fig. 3 Teil dieses Dokuments sind.

Eine Dosiervorrichtung 1 gemäß Fig. 1 weist einen als Drucktank 2 ausgeführten Mediumspeicher sowie eine Mediumkontrolleinrichtung 24 auf. Der Drucktank 2 enthält insbesondere in einem Glasbehälter 22 eine hermetisch abgeschlossene Menge Flüssigmedium 23. Das Flüssigmedium 23 wird durch einen Druckkolben 20, der von einer Federeinrichtung 21 mit einer Druckkraft beaufschlagt wird, unter hydrostatischen Druck gesetzt. Die Mediumkontrolleinrichtung 24 weist einen als Verbindungsrohr 3 ausgeführten Mediumkanal und eine als nicht näher dargestellte Düse 4 ausgeführte Austragöffnung auf. Weiterhin ist an der Mediumkontrolleinrichtung eine als Regelventil 9 ausgeführte Stelleinrichtung und eine als Volumenstrommesser 6 ausgeführte Messeinrichtung vorgesehen. Der Volumenstrommesser 6 und das Regelventil 9 sind dabei über eine als integrierte Schaltung 5 ausgeführte Regeleinheit gekoppelt. Die integrierte Schaltung 5 wird mit einem Eingangssignal 25 des Volumenstrommessers 6 versorgt und erzeugt ein Ausgangssignal 26 zur Ansteuerung des Regelventils 9. Dafür wird das Eingangssignal 25 als Istwert in einem Speicher 7 der Schaltung 5 zeitweilig abgelegt und nachfolgend in einem Vergleicher 8 mit einem Sollwert aus einem Sollwertregister 19 verglichen. Aus diesem Vergleich folgt das Ausgangssignal 26, das zur Beeinflussung des Regelventils 9 vorgesehen ist.

Für einen Mediumaustrag wird über nicht dargestellte Auslösemittel ein Austragsignal an die integrierte Schaltung 5 gesandt, das dort einen ersten Sollwert aus dem Sollwertregister 19 in den Vergleicher 8 eintreten lässt. Da zu diesem Zeitpunkt kein Austragvorgang stattfindet, erkennt der Vergleicher 8 eine große Differenz zwischen dem Sollwert und dem aus dem Eingangssignal 25 ermittelten Istwert und löst ein entsprechendes Ausgangssignal 26 zur Ansteuerung des Regelventils 9 aus. Durch dieses Ausgangssignal 26 öffnet das Regelventil 9 und ermöglicht so ein Ausströmen des Flüssigmediums 23 aus dem Drucktank 2 vorbei an dem Volumenstrommesser 6 in die Düse 4. Von dort tritt das Medium in eine Umgebung der Dosiervorrichtung aus. Während des Vorbeiströmens an dem Volumenstrommesser 6 wird ein neues Eingangssignal 25 hervorgerufen, das an den Speicher 7 der integrierten Schaltung 5 weitergegeben wird. Dieses Eingangssignal wird mit dem entsprechenden Sollwert aus dem Sollwertregister 19 im Vergleicher 8 abgeglichen und führt zu einem neuerlichen Ausgangssignal 26. Durch diesen geschlossen Regelkreis kann ein Mediumaustrag anhand vorgegebener Sollwerte präzise dosiert werden.

In Fig. 2 ist eine der Mediumkontrolleinrichtung 24 nachschaltbare Zerstäubungseinrichtung 32 dargestellt, die einen als Ultraschallkammer 10 ausgeführten Dosierraum sowie eine als Steuergerät 13 verwirklichte Abgabefunktionseinheit aufweist. Dabei ist der Ultraschallkammer 10 eine als Ultraschallschwinger 11 ausgeführte Vibrationseinheit zugeordnet. Der Ultraschallschwinger 11 ist an einer als Substrat 12 vorgesehenen Begrenzungsfläche angebracht und kann über ein Stellsignal 30 von dem Steuergerät 13 mit einer elektrischen Spannung zur Aktivierung versorgt werden. Die Ultraschallkammer 10 ist weiterhin durch eine Membran 28 begrenzt, in der mehrere als Düsen 4 vorgesehene Austragöffnungen eingebracht sind. Eine Versorgung der Ultraschallkammer 10 mit Flüssigmedium 23 ist über einen Einströmkanal 33 sowie über eine als Mäanderspeicher 14 ausgeführte Vorratskammer vorgesehen. Der Mäanderspeicher 14 ist mit einer als Volumenmesser 6 ausgeführten Messeinrichtung versehen, die ein Messsignal 29 an das Steuergerät 13 abgibt. Vom Steuergerät 13 wird ein Synchronisationssignal an die nicht dargestellte integrierte Schaltung 5 der Mediumkontrolleinrichtung 24 weitergeleitet, um eine Funktion der Zerstäubungseinrichtung 32 mit der Mediumkontrolleinrichtung 24 zu synchronisieren.

Für einen Austragvorgang wird ein Synchronisationssignal 31 von der nicht dargestellten integrierten Schaltung 5 an das Steuergerät 13 abgesandt. Dadurch wird in Stromrichtung 27 ein Mediumstrom von der nicht dargestellten Mediumkontrolleinrichtung in den Einströmkanal 33 abgegeben. Durch das Synchronisationssignal 31 wird im Steuergerät 13 ein Stellsignal 30 erzeugt, das auf den Ultraschallschwinger 11 wirkt und diesen zur Ausübung von Schwingungsbewegungen aktiviert. Durch die Schwingungsbewegungen wird das in die Ultraschallkammer 10 eingeströmte Medium durch eine Reduzierung des Volumens der Ultraschallkammer 10 zumindest zeitweilig unter Druck gesetzt und kann entgegen dem Strömungswiderstand der Düsen 4 in die Umgebung abgegeben werden. Da der Ultraschallschwinger 11 die Ultraschallkammer 10 mit einer insbesondere periodischen Schwingung beaufschlagt, kommt es unmittelbar nach dem erfolgten zeitweiligen Mediumaustrag durch die Düsen 4 wieder zu einer Vergrößerung des freien Volumens in der Ultraschallkammer. Dadurch kann insbesondere durch Kapillarkräfte Medium aus dem Mäanderspeicher 14 in die Ultraschallkammer 11 nachströmen. Aus dem Einströmkanal 33 erfolgt kein Nachströmen von Medium, da der Einströmkanal 33 durch das in Fig. 1 bzw. Fig. 4 dargestellte Regelventil 6 verschlossen ist. Das Nachströmen von Medium aus dem Mäanderspeicher 14 führt gemäß Fig. 2 zu einer Signaländerung an dem Volumenmesser 6, der im Mäanderspeicher 14 vorgesehen ist. Das entsprechende Messsignal 29 wird an das Steuergerät 13 weitergeleitet und dort in ein Gesamtsignal zur Kennzeichnung eines ausgetragenen Volumenstroms umgerechnet. Mit vollständiger Entleerung des Mäanderspeichers 14 wird der Austragvorgang durch Rückkopplung des Synchronisationssignals 31 an die nicht dargestellte integrierte Schaltung 5 beendet.

Bei der in Fig. 3 dargestellten erfindungsgemäßen Zerstäubungseinrichtung 32 ist im Unterschied zum Ausführungsbeispiel gemäß der Fig. 2 eine stromabwärts nach der Membran 28 als Lichtschranke 15 ausgeführte Sensoranordnung vorgesehen, die ein Messsignal 29 an eine Integrationseinheit 34 abgibt. Die Integrationseinheit 34 ist mit dem Steuergerät 13 elektrisch gekoppelt und ermöglicht die Übermittlung eines Messwertes an das Steuergerät. Dieser Messwert wird in dem Steuergerät 13 in ein Stellsignal 30 für den Ultraschallschwinger 11 umgesetzt, der analog zur Ausführungsform der Fig. 2 die Ultraschallkammer 10 zu Schwingungen anregen kann. Die Lichtschranke 15 ermittelt anhand eines Transmissionswertes die absolut ausgetragene Mediummenge, die an die Steuereinheit und die nicht dargestellte integrierte Schaltung 5 weitergeleitet wird.

## Patentansprüche

1. Dosiervorrichtung (1) für ein Medium mit einem Mediumspeicher (2), mit wenigstens einem Mediumkanal (3) zur Förderung des Mediums (23) und mit zumindest einer Austragöffnung (4) zum Ausbringen des Mediums (23), wobei dem Mediumkanal (3) eine Regeleinheit (5) zugeordnet ist, die eine Meßeinrichtung (6) zur Erfassung eines Istwertes eines Mediumstroms, einen Speicher (7) für Sollwerte des Mediumstroms, einen Vergleicher (8) zwischen Ist- und Sollwerten und eine Stelleinrichtung (9) zur Beeinflussung des Mediumstroms abhängig von Auswertungen des Vergleichers (8) aufweist,
**dadurch gekennzeichnet, dass**
die Meßeinrichtung (6) in Fließrichtung des Mediums stromabwärts nach der Austragöffnung (4) angeordnet ist.

2. Dosiervorrichtung nach Anspruch 1, **dadurch gekennzeichnet, dass** an dem Mediumkanal (3) ein Dosierraum (10) zur zumindest zeitweisen Aufnahme einer Flüssigkeitsmenge vorgesehen ist, dem die wenigstens eine Austragöffnung (4) zugeordnet ist, wobei der Dosierraum (10) mit einer Vibrationseinheit (11) versehen ist, die wenigstens mit einer Begrenzungsfläche (12) des Dosierraums (10) in Wirkverbindung steht, um diese für einen Austragvorgang in Schwingungen zu versetzen, wobei eine mit der Vibrationseinheit (11) verbundene Abgabefunktionseinheit (13) zur Aktivierung der Vibrationseinheit während einer Abgabezeitdauer vorgesehen ist.

3. Dosiervorrichtung nach Anspruch 2, **dadurch gekennzeichnet, dass** die Abgabefunktionseinheit (13) mit der Regeleinheit (5) zur kontrollierten Mediumabgabe gekoppelt ist.

4. Dosiervorrichtung nach einem der vorhergehenden Ansprüche, **dadurch gekennzeichnet, dass** die Meßeinrichtung (6) eine optische und/oder kapazitive und/oder induktive Sensoranordnung (15) aufweist.

5. Dosiervorrichtung nach einem der vorhergehenden Ansprüche, **dadurch gekennzeichnet, dass** der Mediumspeicher (2) druckbeaufschlagt ist.

6. Dosiervorrichtung nach einem der vorhergehenden Ansprüche, **dadurch gekennzeichnet, dass** in dem Mediumkanal (3) und/oder dem Mediumspeicher (2) ein Gasabscheider vorgesehen ist.

7. Dosiervorrichtung nach Anspruch 6, **dadurch gekennzeichnet, dass** an dem Mediumspeicher (2) und/oder dem Gasabscheider ein, insbesondere als hydrophober Membranfilter ausgeführter, Gasauslass vorgesehen ist.

## Claims

1. Dosing device (1) for a medium, with a medium reservoir (2), with at least one medium channel (3) for delivering the medium (23) and with at least one dispensing opening (4) for dispensing the medium (23), the medium channel (3) being assigned a control unit (5) which comprises a measurement means (6) for detecting an actual value of a stream of medium, a memory (7) for desired values of the stream of medium, a comparator (8) for comparison between actual values and desired values, and an actuating means (9) for acting on the stream of medium as a function of evaluations performed by the comparator (8),
**characterized in that**
the measurement means (6) is arranged in a flow direction of the medium downstream subsequent to the dispensing opening (4).

2. Dosing device according to claim 1, **characterized in that** a dosing chamber (10) is provided on the medium channel (3) for at least temporarily receiving a quantity of liquid and is assigned the at least one dispensing opening (4), wherein the dosing chamber (10) is provided with a vibration unit (11) which is operatively connected at least to one boundary surface (12) of the dosing chamber (10) in order to cause this boundary surface to oscillate for the purpose of a dispensing operation, and wherein an output function unit (13) connected to the vibration unit (11) is provided for activating the vibration unit during an output period.

3. Dosing device according to claim 2, **characterized in that** the output function unit (13) is coupled to the control unit (5) for controlled output of medium.

4. Dosing device according to any one of the preceding claims, **characterized in that** the measurement means (6) includes an optical and/or capacitive and/or inductive sensor array (15).

5. Dosing device according to any one of the preceding claims, **characterized in that** the medium reservoir (2) is pressurized.

6. Dosing device according to any one of the preceding claims, **characterized in that** a gas separator is provided in the medium channel (3) and/or in the medium reservoir (2).

7. Dosing device according to claim 6, **characterized in that** a gas outlet is provided on the medium reservoir (2) and/or on the gas separator, in particular in the configuration of a hydrophobic membrane filter.

## Revendications

1. Dispositif de dosage (1) pour une substance, comprenant un réservoir de substance (2), comprenant au moins un canal de substance (3) pour l'acheminement de la substance (23), et comprenant au moins une ouverture de distribution (4) pour la distribution de la substance (23), une unité de régulation (5) étant associée au canal de substance (3), laquelle comprend un dispositif de mesure (6) pour détecter une valeur réelle d'un débit de substance, une mémoire (7) pour des valeurs de consigne du débit de substance, un comparateur (8) entre les valeurs réelles et de consigne et un dispositif de réglage (9) pour influencer le débit de substance en fonction d'évaluations du comparateur (8),
**caractérisé en ce que**
le dispositif de mesure (6) est disposé en aval après l'ouverture de distribution (4) dans le sens d'écoulement de la substance.

2. Dispositif de dosage selon la revendication 1, **caractérisé en ce qu'**un espace de dosage (10) pour recevoir au moins temporairement une quantité de liquide est prévu sur le canal de substance (3), auquel espace de dosage est associée au moins une ouverture de distribution (4), l'espace de dosage (10) étant pourvu d'une unité de vibration (11) qui est en liaison fonctionnelle au moins avec une surface de limitation (12) de l'espace de dosage (10), afin de mettre en vibrations celle-ci pour une opération de distribution, une unité fonctionnelle de distribution (13) reliée à l'unité de vibration (11) étant prévue pour l'activation de l'unité de vibration pendant une durée de distribution.

3. Dispositif de dosage selon la revendication 2, **caractérisé en ce que** l'unité fonctionnelle de distribution (13) est accouplée à l'unité de régulation (5) pour la distribution contrôlée de substance.

4. Dispositif de dosage selon l'une quelconque des revendications précédentes, **caractérisé en ce que** le dispositif de mesure (6) comprend un ensemble de capteur (15) optique et/ou capacitif et/ou inductif.

5. Dispositif de dosage selon l'une quelconque des revendications précédentes, **caractérisé en ce que** le réservoir de substance (2) est sollicité en pression.

6. Dispositif de dosage selon l'une quelconque des revendications précédentes, **caractérisé en ce qu'**un séparateur de gaz est prévu dans le canal de substance (3) et/ou dans le réservoir de substance (2).

7. Dispositif de dosage selon la revendication 6, **caractérisé en ce qu'**une sortie de gaz réalisée en particulier sous forme de membrane filtrante hydrophobe est prévue sur le réservoir de substance (2) et/ou le séparateur de gaz.
